(19) Europäisches Patentamt

European Patent Office

Office européen des brevets

(11) **EP 0 914 832 B1**

(12) **EUROPEAN PATENT SPECIFICATION**

(45) Date of publication and mention
of the grant of the patent:
**22.11.2006 Bulletin 2006/47**

(21) Application number: **98911200.8**

(22) Date of filing: **02.04.1998**

(51) Int Cl.:
***A61K 47/36*** (2006.01)    ***A61K 9/00*** (2006.01)

(86) International application number:
**PCT/JP1998/001549**

(87) International publication number:
**WO 1998/044950 (15.10.1998 Gazette 1998/41)**

(54) **THERAPEUTIC MATERIAL FOR PERIODONTOSIS**

THERAPEUTISCHES MATERIAL FÜR PERIODONTITIS

MATERIAU THERAPEUTIQUE POUR LA PARODONTOSE

(84) Designated Contracting States:
**DE FR GB SE**

(30) Priority: **04.04.1997 JP 10268297**

(43) Date of publication of application:
**12.05.1999 Bulletin 1999/19**

(73) Proprietor: **KURARAY CO., LTD.**
**Kurashiki-shi, Okayama 710-8622 (JP)**

(72) Inventor: **YAMADA, Hideaki**
**Kurashiki-shi**
**Okayama 710-8622 (JP)**

(74) Representative: **Schüssler, Andrea et al**
**Kanzlei Huber & Schüssler**
**Truderinger Strasse 246**
**81825 München (DE)**

(56) References cited:
**EP-A- 0 459 733        EP-A- 0 512 855**
**EP-A- 0 712 635        WO-A-96/13253**
**JP-A- 3 287 538        JP-A- 8 024 325**
**JP-A- 62 158 701       JP-T- 7 502 431**

**Description**

TECHNICAL FIELD

The present invention relates to a material for treating periodontal disease for regenerating periodontal tissues lost by periodontal disease and to the production of a medicament for treating periodontal disease using the material for treating periodontal disease.

BACKGROUND ART

Conventionally, periodontal disease has been treated by periodontal pocket curettage, which comprises curetting and removing the affected portion, disinfecting the surrounding tissues, and suturing the surgical wound. However, in this method, there arises a problem that since gingival epithelial cells migrate into periodontal pockets (down growth) after treatment, the growth of periodontal cells is suppressed, and connective tissues bind directly to the root surface, thereby resulting in the interference of normal tissue regeneration.

In view of the above, guided tissue regeneration (GTR) has recently been developed and has been markedly noted as a method of treatment for regeneration of periodontal tissues lost due to the periodontal disease. Nyman et al. demonstrated that periodontal cells are proliferated on the root surface to achieve new attachment of newly formed bone by isolating the root portion from the epithelial tissues, connective tissues, and the like, using Millipore filters [*Journal of Clinical Periodontology*, 9, 290-296 (1982)]. Gottlow et al. similarly demonstrated a possibility of periodontal tissue regeneration using Gore-tex membranes [*Journal of Clinical Periodontology*, 11, 494-503 (1984)]. Because all such membranes are not bioabsorbable, however, there arises the problem of the necessity of secondary surgery for membrane removal after periodontal tissue regeneration.

In order to solve this problem, some GTR membranes using bioabsorbable materials have been developed. Japanese Patent Laid-Open No. Hei 2-63465 discloses a membrane comprising a lactic acid/glycolic acid copolymer; Japanese Patent Laid-Open No. Hei 3-294209 discloses a membrane comprising atelocollagen; and Japanese Patent Laid-Open Nos. Hei 4-134035 and Hei 4-285565 each discloses a membrane comprising chitin. Although all these membranes are bioabsorbable and secondary surgery for membrane removal is not necessitated, high skill and great care are required to optimally cover the alveolar bone resorption portion owing to a lack of the strength of the membrane itself. In addition, these membranes are difficult to apply for cases of high resorption of alveolar bone and in cases of affected portions with complicated shapes. Furthermore, because the membrane is likely to deform during treatment, epithelium migrates into periodontal pockets, thereby interfering with tissue regeneration in some cases. When the mechanical strength of the membrane is increased for the purpose of preventing deformation, the membrane is exposed through the epithelium during treatment, which in turn causes the problem of bacterial infection.

In order to solve the above-described problems in the membrane-type GTR materials, Japanese Patent Laid-Open No. Hei 4-99725 discloses paste-like agents for promoting periodontal tissue regeneration comprising an aliphatic polyester, such as a glycolic acid/lactone copolymer, as GTR materials that can replace membrane-type materials. These agents are intended to secure a site for periodontal tissue regeneration by injecting a paste-like material in the periodontal tissue resorption portion, using a syringe etc. However, because of insufficient blood penetration into the packed paste, there arises a problem of delayed regeneration of periodontium. Also, because a gap is likely to be formed between the packed paste and the tooth, there yet remains another problem to be solved of interference of tissue regeneration owing to the fact that the epithelial cells migrate into periodontal pockets through the gap.

Japanese Patent Laid-Open No. Hei 3-287538 discloses a gel-type material for treating periodontal disease comprising a polymeric electrolyte complex of a cationic polymer and an anionic polymer, and glycerol added thereto. Similar to the paste-like material, this material poses the problem of delayed regeneration of periodontal tissue because of insufficient blood penetration in the packed gel.

Furthermore, all the above-described bioabsorbable materials have been reported to have various problems in terms of biocompatibility. For example, the glycolic acid/lactone copolymer and chitin both pose the problem of inflammation-provoking potential, and atelocollagen poses the problem of sensitization due to its antigenicity. In order to solve these problems, more biocompatible materials have been in demand. Japanese Unexamined Patent Publication No. Hei 7-502431 discloses membrane-type GTR materials comprising a polysaccharide, such as alginic acid or hyaluronic acid. These polysaccharides possess remarkably excellent biocompatibility so that they have no problems in terms of infectivity and sensitization as those of the bioabsorbable materials. However, it is an essential requirement for them to be used in membranous form, and the drawback of poor operability of the membrane-type GTR materials remains unresolved. Japanese Patent Laid-Open No. Hei 8-24325 discloses a water-swellable polymer gel obtained by crosslinking an acidic polysaccharide, such as alginic acid or hyaluronic acid, with a diamine. However, this publication does not describe a use of the polymer gel as a GTR material for treating periodontal disease.

WO 96/13253 A describes the production of controlled release microcapsules of alginate coated with chitosan, wherein a drug for treating periodontitis is incorporated therein. Furthermore, pharmaceutical uses of cross-linked alginates, e.g. wound fillers and wound dressings, are disclosed in EP 0459733 A, EP 0512855 A and EP 0712635 A.

Therefore, a first object of the present invention is to provide a medicament for treating periodontal disease in which the problems conventionally owned by the membrane-type or paste-like materials for treating periodontal disease such as poor operability, difficulty in applying to periodontal pockets with complicated shapes, deficient mechanical strength, and delay in tissue regeneration have been solved. Further, a second object of the present invention is to provide a medicament for treating periodontal disease which can induce alveolar bone regeneration. These and other objects of the present invention will be apparent from the following description.

DISCLOSURE OF INVENTION

As a result of intensive study on the means of solving the above problems, the present inventors have found that the above problems can be solved by using a dry gel of a polysaccharide which is crosslinked by using a crosslinking agent. In other words, the inventors have found that the use of the dry gel has the advantages of more easily packing even into a periodontal pocket with a complicated shape as compared to a membrane-type material for treating periodontal disease; when the dry gel of the polysaccharide which is crosslinked by using a crosslinking agent is inserted into the periodontal pocket, the gel fills up in the periodontal pocket by absorbing blood and tissue fluids in the pocket and swelling thereby, so that a space for periodontal tissue regeneration filled with blood and tissue fluids can be secured, thereby inducing tissue regeneration; and further the swelled gel has sufficient mechanical strength so as not to cause down growth of the epithelial cells. The present invention has been completed based on these findings.

In sum, the present invention pertains to:

the use of a dry gel of a polysaccharide which is crosslinked by using a crosslinking agent, for the production of a medicament for treating periodontal disease.

BEST MODE FOR CARRYING OUT THE INVENTION

The present invention will be described in detail below.

1. Medicament for treating periodontal disease of the present invention

The medicament for treating periodontal disease of the present invention comprises a dry gel of a polysaccharide which is crosslinked by using a crosslinking agent.

The term "medicament for treating periodontal disease" as used in the present invention refers to a material usable to secure a space for periodontal tissue regeneration and to prevent the down growth of epithelium, by packing the material in a periodontal pocket, with the purpose of regeneration of periodontal tissues lost due to the periodontal disease. The medicament for treating periodontal disease of the present invention may consist only of a dry gel of a polysaccharide which is crosslinked by using a crosslinking agent, or it may be a composite material of the dry gel and another material, such as a dry gel of another bioabsorbable polymer, such as a polylactic acid, a polyglycolic acid, a lactic acid/glycolic acid copolymer, collagen, or gelatin.

In addition, for the purposes of controlling the water content and imparting tackiness, the dry gel may be supplemented with pharmacologically acceptable additives, including inorganic ions, such as $Na^+$, $Ca^{2+}$ and $Mg^{2+}$; polyhydric alcohols, such as ethylene glycol, propylene glycol, glycerol and PEG; and polymeric compounds, such as polyvinyl alcohols and polyacrylic acids.

As the crosslinked polysaccharide used in the present invention, any of the crosslinked polysaccharides can be used as long as they can be prepared by crosslinking functional groups carried by the polysaccharide, such as carboxyl groups, amino groups and hydroxyl groups, by using a crosslinking agent. The crosslinked polysaccharides include, for instance, those prepared by crosslinking acidic polysaccharides, such as alginic acid, hyaluronic acid, pectin, carboxymethyl cellulose, carboxymethyl dextran, dextran sulfate and chondroitin sulfate; basic polysaccharides, such as chitosan and aminocellulose; neutral polysaccharides, such as cellulose, starch and dextran; derivatives of these polysaccharides; and salts of these polysaccharides or derivatives thereof. From the viewpoint of increasing tooth adhesion, those prepared by crosslinking acidic polysaccharides are preferred, with greater preference given to those prepared by crosslinking one or more compounds selected from the group consisting of alginic acid, alginic acid derivatives, and salts of alginic acid or alginic acid derivatives. The term "polysaccharide" as used in the present specification is understood to encompass polysaccharides, derivatives thereof, and salts thereof.

Examples of the polysaccharide derivative include those prepared by reacting some or all of the hydroxyl groups carried by the polysaccharide with acetic acid, nitric acid, sulfuric acid, phosphoric acid, or the like; and those prepared by esterifying some of the carboxyl groups carried by an acidic carboxyl group-carrying polysaccharide with a low molecular alcohol, such as ethylene glycol or propylene glycol, with preference given to carboxymethyl cellulose acetate, carboxymethyl dextran acetate, ethylene glycol alginate, propylene glycol alginate, ethylene glycol hyaluronate and propylene glycol hyaluronate. Although the degree of esterification in the compounds resulting from esterification of the above acidic polysaccharides is not limited to specified ones, it is preferable that the degree of esterification is not higher than 80%, more preferably not higher than 30%. This is because when the degree of esterification is too high, the ratio of carboxyl groups which can be used for crosslinking is lowered, so that the mechanical strength of the gel formed from the crosslinked product of the derivative tends to be decreased. In addition, as to salts, there can be favorably used salts formed between acidic polysaccharides or derivatives thereof and monovalent ions, exemplified by alkali metal salts, such as sodium salts and potassium salts; ammonium salts, and the like. Examples of preferable salts with alginic acid include sodium salts.

Alginic acid is contained in large amounts in brown algae, such as seaweed wakame and kelp, and comprises 1,4'-bond residues of D-mannuronic acid and L-guluronic acid. The alginic acid used in the present invention is preferably extracted from these brown algae, from the viewpoint of obtaining the alginic acid readily and in large amounts. Although there are different alginic acids with different mannuronic acid/guluronic acid compositional ratios depending on the kinds of brown algae and the methods of extraction, any form of alginic acid can be used regardless of the compositional ratio in the present invention.

Among the different kinds of alginic acid, those having a viscosity at 20°C of not lower than 100 cp (centipoise), when prepared as 1% by weight aqueous sodium alginate solution, is preferred from the viewpoint of an alginic acid gel strength, and the like, with greater preference given to those having the above viscosity of not lower than 300 cp. Since excess viscosity makes the production of an alginic acid gel difficult, however, the alginic acid having the above viscosity not higher than 1,000 cp are preferred.

The crosslinked polysaccharide in the present invention is prepared by crosslinking polysaccharides by using a crosslinking agent. Modes for crosslinking include crosslinking by covalent bonds and crosslinking by ionic bonds, with preference given to the crosslinking by covalent bonds from the viewpoint of imparting gel strength. In addition, examples of the crosslinking agent include polyvalent reactive compounds, such as compounds having a total of two or more functional groups selected from amino groups, carboxyl groups, aldehyde groups, and the like; and the salts of divalent or higher metal ions, such as calcium ions and magnesium ions. Methods for crosslinking a polysaccharide are described below.

In the case of an acidic polysaccharide having carboxyl groups, such as alginic acid, hyaluronic acid, pectin, carboxymethyl cellulose or carboxymethyl dextran, carboxyl group of the acidic polysaccharide and amino group of a crosslinking agent is bound via a covalent bond by a dehydration condensation reaction using a diamine and/or a polyamine as a crosslinking agent.

Specifically, crosslinking agents are exemplified by the salts of diaminoalkanes, such as diaminoethane, diaminopropane, diaminobutane, diaminopentane, diaminohexane, diaminoheptane, diaminooctane, diaminononane, diaminodecane, diaminododecane and diaminooctadecane; and the salts of mono- or di(lysyl)diaminoalkanes, such as N-(lysyl)-diaminoethane, N,N'-di(lysyl)-diaminoethane, N-(lysyl)-diaminohexane and N,N'-di(lysyl)-diaminohexane. The 2N-hydroxysuccinimide salt of diaminoethane, the 2N-hydroxysuccinimide salt of diaminohexane, the 4N-hydroxysuccinimide salt of N,N'-di(lysyl)-diaminoethane, the 3N-hydroxysuccinimide salt of N-(lysyl)-diaminohexane, and the like are particularly preferred. Also, the polysaccharide can be gelated by formation of crosslinking by ionic bonds by adding calcium chloride to an aqueous solution of an acidic polysaccharide having carboxyl groups.

In the case of a basic polysaccharide having amino groups, such as chitosan and aminocellulose, amino group of the basic polysaccharide and carboxyl group of a crosslinking agent is bound via a covalent bond by a dehydration condensation reaction using a dicarboxylic acid and/or a polycarboxylic acid as a crosslinking agent. Preferable crosslinking agents include anhydrides of dicarboxylic acids, such as succinic anhydride and maleic anhydride. Also, a Schiff base is formed between amino group of the basic polysaccharide and aldehyde group of a crosslinking agent by using a dialdehyde, such as glutaraldehyde, as a crosslinking agent, so that crosslinking via covalent bonds can be achieved.

In the case of a polysaccharide carrying neither carboxyl groups nor amino groups, such as cellulose, starch, dextran, dextran sulfate and chondroitin sulfate, crosslinking can be achieved in the same manner as the acidic polysaccharides having carboxyl groups or the basic polysaccharides having amino groups, after carboxyl groups or amino groups are introduced by utilizing hydroxyl groups contained therein. The method for introducing amino groups by utilizing hydroxyl groups includes a process comprising epoxidizing hydroxyl group with epichlorohydrin, and then reacting the resulting epoxidized compound with aqueous ammonia. Subsequently, carboxyl group can be introduced by further reacting an acid anhydride, such as succinic anhydride, with the resulting compound in the presence of a base, such as pyridine.

From the viewpoint of the mechanical strength of the gel upon water absorption and its swelling in a periodontal pocket, it is preferable that the degree of crosslinking for the crosslinked polysaccharide in the present invention is such that not less than 1%, more preferably not less than 5%, of a total number of the monosaccharides constituting the polysaccharide are crosslinked. When the mechanical strength of the gel is insufficient, the down growth of epithelial cells cannot be prevented. Also, the degradation and absorption rates in the periodontal pocket become too high, so that the gel disappears before periodontal tissue regeneration, thereby making it inappropriate. On the other hand, from the viewpoint of volume increase rate for the gel upon water absorption and swelling in the periodontal pocket, the ratio of the crosslinked monosaccharides is preferably not more than 75%, more preferably not more than 50%. When the volume increase rate of the gel is too small, it is difficult to pack the periodontal pocket closely. Also, since bioabsorption rates are delayed, the gel is likely to remain in the tissue even after periodontal tissue regeneration, thereby making it inappropriate. Therefore, in the crosslinked polysaccharide usable in the present invention, it is preferable that 1 to 75%, more preferably from 5 to 50%, of a total number of the monosaccharides constituting the polysaccharide are crosslinked.

The degree of crosslinking can be controlled by a molar ratio of the crosslinking agent to the polysaccharide, and can be measured by elementary analysis, NMR, and the like. For example, in a case where a polysaccharide without containing any nitrogen atoms, such as an alginate or hyaluronate, is crosslinked with a diamine and/or a polyamine, the degree of crosslinking can be determined by elementary analysis for the nitrogen atoms in the gel obtained. It can also be determined from the signal intensity ratio of the methylene proton of the polysaccharide and the methylene proton of the crosslinking agent in proton NMR for the gel obtained.

The material for treating periodontal disease of the present invention is used in a dry state, i.e., in the form of a dry gel.

A use of the gel in a dry state has the advantages that 1) since surrounding blood and tissue fluid are penetrated, tissue regeneration is promoted; and that 2) since the gel is swelled at the site of application, the periodontal pocket can be packed closely.

As the method for drying the crosslinked polysaccharide, any methods can be employed as long as the polysaccharide is not deteriorated or otherwise changed. For example, known methods which are conventionally employed, such as lyophilizing method, spray drying method, method of drying under reduced pressure, solvent-replacement drying method, method of drying with heating, and method of drying with warm air, can be used. Most preferably, the lyophilizing method is appropriately used. In other words, a method comprising dissolving a polysaccharide in water or another solvent, then freezing, and drying under reduced pressure is appropriately used.

Although the degree of drying for the dry gel usable in the present invention is not limited to specified ones, the water concentration in the dry gel is preferably from 0 to 10% by weight, more preferably from 0 to 1% by weight.

From the viewpoint of securing a sufficient space for periodontal tissue regeneration and preventing the epithelial down growth, it is preferable that the amount of water absorption by the dry gel of the crosslinked polysaccharide usable in the present invention ranges from 5 to 50 g, more preferably from 10 to 30 g, per gram of the dry gel. The amount of water absorption can be determined as the amount of water retained in the gel when water is added to 1 g of the dry gel.

The medical material for treating periodontal disease of the present invention is not subject to limitation with respect to shape, structure, etc., as long as the material is supplied in a dry state. For example, it can be used in the forms of sponges, nets, unwoven fabrics, woven knittings, particles, powders, flakes, and the like. In order to pack a periodontal pocket of a complicated shape closely, it is preferable that the medicament for treating periodontal disease is used in a powdery state of a size of a 140 mesh sieve-pass after being finely milled.

The medicament for treating periodontal disease of the present invention can be used alone, or it can also be used in combination with another treatment material. For example, by a combined use with a membrane-type GTR material, the collapse of the membrane-type material into the periodontal pocket owing to deficient mechanical strength of the membrane-type material for treating periodontal disease can be prevented.

As described above, according to the present invention, there is provided a use of a dry gel of a polysaccharide which is crosslinked by using a crosslinking agent, for the production of a medicament for treating periodontal disease.

2. Use of the medicament for Treating Periodontal Disease of the Present Invention

The use of the medicament for treating periodontal disease of the present invention is characterized by inserting a dry gel of a polysaccharide which is crosslinked by using a crosslinking agent into a resorption portion of alveolar bone lost due to periodontal disease in an amount sufficient to induce alveolar bone regeneration.

Specifically, this includes a process comprising peeling the epithelium around a tooth affected by periodontal disease, curetting and removing the infected portion, then subjecting it to root planing and sterilization, forming a periodontal pocket, and inserting a dry gel of a crosslinked polysaccharide into the resulting periodontal pocket. When inserted into a periodontal pocket, the medicament for treating periodontal disease of the present invention absorbs and swells with the blood and tissue fluid in the periodontal pocket, and packs the pocket, which results in the securing of a space for periodontal tissue regeneration packed with blood and tissue fluid, so that the tissue regeneration is induced.

The amount of the dry gel of a crosslinked polysaccharide used may be preferably that sufficient to induce alveolar bone regeneration.

In this method for treating periodontal disease, since the gel swells in the periodontal pocket, the pocket is packed closely. Adhesion to the tooth is, therefore, enhanced, so that the epithelial down growth can be prevented and a space for periodontal tissue regeneration can be secured. Because the swollen gel is gradually decomposed and absorbed as periodontal tissues are concurrently regenerated, and eventually disappears, surgery for its removal after healing is unnecessary.

Also, since the medicament for treating periodontal disease of the present invention can easily pack in periodontal pockets of a complicated shape without highly skilled techniques as required for membrane-type GTR materials, surgery operation can be carried out in a simple manner.

As described above, the present invention provides a use of the medicament for treating periodontal disease of the present invention for the treatment of periodontal disease.

The present invention is hereinafter described in more detail by means of the following working examples, comparative examples and test example, without intending to limit the scope of the present invention thereto.

Example 1

In 150 ml of methanol was dissolved 2.3 g of N-hydroxysuccinimide (manufactured by Peptide Institute, Inc.). To this solution was added dropwise a solution which was prepared by dissolving 0.6 g of ethylenediamine (manufactured by Wako Pure Chemical Industries, Ltd.) in 10 ml of methanol, with stirring at room temperature. After completion of the dropwise addition, the mixture was continued stirring for additional one hour. The precipitated crystals were collected by filtration and dried under reduced pressure to give 2.6 g of a 2N-hydroxysuccinimide salt of ethylenediamine (yield: about 90%).

In 100 ml of distilled water was dissolved 1 g of sodium alginate (manufactured by Wako Pure Chemical Industries, Ltd.; viscosity when prepared as a 1% by weight-aqueous solution at 20°C: 450-600 cp). Thereafter, in the resulting solution were dissolved 220 mg of the 2N-hydroxysuccinimide salt of ethylenediamine prepared above and 1.6 g of 1-ethyl-3-(3-dimethylaminopropyl)-carbodiimide hydrochloride (manufactured by Peptide Institute, Inc.). The obtained solution was spread over a Teflon-coated tray and kept standing at 25°C for 48 hours to form an alginic acid gel.

The resulting alginic acid gel was washed with water for injection (manufactured by Otsuka Pharmaceutical Co., Ltd.) in which 2.5 mM calcium chloride and 143 mM sodium chloride were dissolved, and then the washed alginic acid gel was further washed with water for injection without calcium chloride and sodium chloride. The alginic acid gel after the washing process was lyophilized, to give a sponge-like lyophilized gel. The lyophilized gel was then milled, and those with a size of 140 mesh sieve-pass were collected.

The powder of the alginic acid gel prepared above was sterilized by γ-ray irradiation at 25 kGy, to prepare a material for treating periodontal disease. The resulting material for treating periodontal disease subjected to a test as described in Test Example. The results are shown in Table 1. The ratio of constituting monosaccharides crosslinked in the resulting alginic acid gel was 25% of the entire constituting monosaccharide content of the alginic acid gel. Here, the ratio is calculated from the ratio of each of the constituting elements determined by elementary analysis.

Example 2

A sponge-like lyophilized gel of the crosslinked alginic acid was prepared in the same manner as in Example 1. The gel was cut into cubular forms with a side length of about 5 mm, and the cubular gel was then sterilized by γ-ray irradiation at 25 kGy, to prepare a medicament for treating periodontal disease.

Example 3

While stirring a 1% by weight aqueous calcium chloride solution, a 1% by weight aqueous sodium alginate solution (manufactured by Wako Pure Chemical Industries, Ltd.; viscosity when prepared as a 1% by weight aqueous solution at 20°C: 450-600 cp) was added dropwise to form an alginic acid gel. The resulting alginic acid gel was washed with water for injection (manufactured by Otsuka Pharmaceutical Co., Ltd.), and the washed alginic acid gel was lyophilized to give a sponge-like lyophilized gel. The lyophilized gel was then milled, and those with a size of 140 mesh sieve-pass were collected. The powder of the alginic acid gel prepared above was sterilized by γ-ray irradiation at 25 kGy, to prepare a medicament for treating periodontal disease.

Example 4

In 100 ml of distilled water was dispersed 1 g of chitosan ("Chitosan 500," manufactured by Wako Pure Chemical Industries, Ltd.), and 3 ml of acetic acid was gradually added dropwise with stirring. The mixture was continued stirring until the chitosan was completely dissolved. To the resulting solution was added dropwise 0.5 ml of glutaraldehyde, and thereafter the resulting solution was spread over a Teflon-coated tray and kept standing at 25°C for 48 hours to form a crosslinked chitosan gel.

The crosslinked chitosan gel was washed with water for injection (manufactured by Otsuka Pharmaceutical Co., Ltd.) in which 2.5 mM calcium chloride and 143 mM sodium chloride were dissolved, and the washed crosslinked chitosan gel was then further washed with water for injection without calcium chloride or sodium chloride. The crosslinked chitosan gel after the washing process was lyophilized, and cut into cubular forms with a side length of about 5 mm, and the cubular chitosan gel was then sterilized by γ-ray irradiation at 25 kGy, to prepare a medicament for treating periodontal disease.

Comparative Example 1

An alginic acid gel was prepared in the same manner as Example 1. The alginic acid gel was washed with water for injection, and thereafter, the washed gel without being subjected to lyophilizing and being water-containing in a paste-like state was packed in an injection syringe, and autoclaved at 121°C for 20 minutes, to prepare a medicament for treating periodontal disease.

Comparative Example 2

In 100 ml of methylene chloride was dissolved 10 g of a lactic acid/glycolic acid copolymer (manufactured by Aldrich; lactic acid:glycolic acid = 50:50; molecular weight: 50000-75000). The resulting solution was spread over a glass plate in a thickness of 0.2 mm, followed by lyophilizing, to prepare a sheet-like membrane. The resulting membrane was then sterilized by γ-ray irradiation at 25 kGy, to prepare a medicament for treating periodontal disease.

Comparative Example 3

In 100 ml of distilled water was dissolved 1 g of sodium alginate (manufactured by Wako Pure Chemical Industries, Ltd.; viscosity when prepared as a 1% by weight aqueous solution at 20°C = 450-600 cp). Onto a slide glass (5 cm x 2 cm) was added dropwise 1 ml of the aqueous sodium alginate solution prepared above. The solution was thinly spread over an entire surface of the slide glass with a stainless steel spatula, and thereafter kept standing overnight to dryness, to prepare a dry film of sodium alginate on the slide glass.

Next, 1 g of chitosan ("Chitosan 500," manufactured by Wako Pure Chemical Industries, Ltd.) was dispersed in 100 ml of distilled water. To this dispersion was gradually added dropwise with stirring 3 ml of acetic acid, and stirring was continued until the chitosan was completely dissolved.

Onto the dry film of sodium alginate prepared above was added dropwise 1 ml of this aqueous solution of the acetic acid salt of chitosan, and thinly spread over the dry film with a stainless steel spatula, and thereafter kept standing overnight to dryness, to prepare a film of a sodium alginate-chitosan polyion complex on the slide glass. The film was peeled off from the slide glass, and the resulting film was then sterilized by γ-ray irradiation at 25 kGy, to prepare a membrane-type medicament for treating periodontal disease.

Comparative Example 4

A sponge-like unwoven fabric of chitin ("BESCHITIN W-A," manufactured by Unitica, Ltd.; uncrosslinked) was tested as described in Test Example as a material for treating periodontal disease.

Test Example

Muco-periosteal flaps were peeled off from the third and fourth premolar teeth on both sides of the mandibles of five healthy mongrel adult dogs. Thereafter, the alveolar bone of about 3 mm thickness was removed from a cement-enamel junction (CEJ) in the direction of the root end, the root furcation was exposed to artificially prepare a root furcation lesion of the second degree, followed by scaling and root planing. The periodontal pockets of two teeth on one side of the mandible were packed with a medicament for treating periodontal disease of Example 1, i.e., a lyophilized powder of a crosslinked alginic acid gel, and the periodontal pockets of two teeth on the other side were packed with a medicament for treating periodontal disease of Comparative Example 1, i.e., a water-containing paste of a crosslinked alginic acid gel, after which each of the periodontal pockets was covered with a gingival flap and sutured.

Similarly, five other mongrel adult dogs were subjected to a test. The periodontal pockets of two teeth on one side of the mandible were packed with a medicament for treating periodontal disease of Example 2, i.e., a lyophilized sponge of a crosslinked alginic acid gel, and the periodontal pockets of two teeth on the other side were packed with a medicament for treating periodontal disease of Comparative Example 2, i.e., a membrane-type lactic acid/glycolic acid copolymer, after which each of the periodontal pockets was covered with a gingival flap and sutured.

In addition, five still other mongrel adult dogs were subjected to a test. The periodontal pockets of two teeth on one side of the mandible were packed with a medicament for treating periodontal disease of Example 3, i.e., a lyophilized powder of a crosslinked alginic acid gel, and the periodontal pockets of two teeth on the other side were packed with a medicament for treating periodontal disease of Comparative Example 3, i.e., a membrane of an alginic acid/chitosan polyion complex, after which each of the periodontal pockets was covered with a gingival flap and sutured.

Further, five still other mongrel adult dogs were subjected to a test. The periodontal pockets of two teeth on one side of the mandible were packed with a medicament for treating periodontal disease of Example 4, i.e., a sponge of a crosslinked chitosan, and the periodontal pockets of two teeth on the other side were packed with a medicament for treating periodontal disease of Comparative Example 4, i.e., sponge-like uncrosslinked chitin, after which each of the periodontal pockets was covered with a gingival flap and sutured.

Twelve weeks after surgery, the animals were sacrificed. Sequential sections of the tissues in the vicinity of the each test tooth (5 sections for each test) were prepared, and subjected to hematoxylin/eosin staining and Azan-Mallory staining. The degree of regeneration of newly formed bone and the state of closure of root furcation were observed for the five sequential sections of each test tooth, and the state of closure was expressed by scores as evaluated by the following standards. The mean scores for the individual samples are shown in Table 1.

Score 0:    No regeneration of newly formed bone is observed, and the root furcation is not closed at all.
Score 1:    Little regeneration of newly formed bone is observed, and the closure of root furcation is incomplete.
Score 2:    Although root furcation is closed completely, newly formed bone does not reach a level of that before alveolar bone removal.
Score 3:    Newly formed bone is regenerated to the level of that before alveolar bone removal, and the root furcation is completely closed.

Furthermore, the depth in which epithelial cell attachment was formed was measured for both the distal and mesial sides of the root of each test tooth, and the epithelial down growth rate was calculated by the equation shown below. The mean values for the individual samples are shown in Table 1.

Also shown in Table 1 are the time required for surgery for carrying out the test and the amount of water absorption by each sample. Incidentally, since the sample for Comparative Example 1 already contained a sufficient amount of water, it could not absorb water any further.

$$\text{Epithelial Down Growth Rate} = \frac{\text{Depth in Which Epithelial Cell Attachment Is Formed}}{\text{Depth at Which Alveolar Bone Is Removed (About 3 mm)}} \times 100(\%)$$

Table 1

| Sample | Material of Sample (Physical Properties) | Mean Score of Regeneration of Newly Formed Bone | Epithelial Down Growth Rate (%) | Amount of Water Absorption (g/g) | Time for Surgery (minutes) |
|---|---|---|---|---|---|
| Examples | | | | | |
| 1 | Crosslinked Alginic Acid Gel (Lyophilized Powder) | 2.9 | 3 | 25 | 12 |
| 2 | Crosslinked Alginic Acid Gel (Lyophilized Sponge) | 2.8 | 5 | 23 | 11 |

(continued)

| Sample | Material of Sample (Physical Properties) | Mean Score of Regeneration of Newly Formed Bone | Epithelial Down Growth Rate (%) | Amount of Water Absorption (g/g) | Time for Surgery (minutes) |
|---|---|---|---|---|---|
| Examples | | | | | |
| 3 | Crosslinked Alginic Acid Gel (Lyophilized Powder) | 2.6 | 8 | 20 | 13 |
| 4 | Crosslinked Chitosan Gel (Lyophilized Sponge) | 2.7 | 9 | 22 | 12 |
| Comparative Examples | | | | | |
| 1 | Crosslinked Alginic Acid Gel (Water-Containing Paste) | 1.2 | 40 | 0 | 18 |
| 2 | Lactic Acid/ Glycolic Acid Copolymer (Membrane) | 2.2 | 35 | 0.2 | 32 |
| 3 | Alginic Acid/ Chitosan Polyion Complex (Membrane) | 2.0 | 42 | 0.2 | 38 |
| 4 | Uncrosslinked Chitin (Sponge) | 1.8 | 25 | 0.5 | 22 |

It is clear from Table 1 that in cases where the medicaments for treating periodontal disease of Examples 1 through 4 were used, all of them had a mean score for the regeneration of newly formed bone ranging from 2.6 to 2.9, and an epithelial down growth rate ranging from 3 to 9%, thereby demonstrating satisfactory regeneration of newly formed bone and mild down growth of epithelium.

In addition, all the medicaments for treating periodontal disease of Examples 1 through 4 could be easily packed in the periodontal pockets, thereby making it possible to complete surgery operation in a short time. In addition, in all of these medicaments for treating periodontal disease of Examples 1 through 4, the packed gel was swelled upon absorption of blood or the like in the periodontal pockets, so that the swelled gel was stably retained therein.

On the other hand, when the medicament for treating periodontal disease of Comparative Example 1, i.e., the water-containing paste of a crosslinked alginic acid, was used, the mean score for the regeneration of newly formed bone was 1.2, and the epithelial down growth rate was 40%, thereby demonstrating poor regeneration of newly formed bone and incomplete closure of the root furcation. This is probably because the epithelial down growth could not be prevented due to an insufficient gel strength owing to the fact that the blood and tissue fluid in the periodontal pocket could not be absorbed upon packing with the gel because of the use of an already swollen paste-like gel, and also because nutrients and growth factors for periodontal tissue regeneration could not be sufficiently supplied.

When the medicament for treating periodontal disease of Comparative Example 2, i.e., a membrane-type lactic acid/glycolic acid copolymer, was used, the mean score for the regeneration of newly formed bone was 2.2, and the root furcation was almost closed; however, the epithelial down growth rate was as high as 35%, because of significant down growth of epithelium owing to membrane deformation at some testing sites. Also, a considerable amount of time was required during surgery operation to completely cover the upper portion of the periodontal pocket owing to the membrane deformation.

Furthermore, when the medicament for treating periodontal disease of Comparative Example 3, i.e., a membrane of an alginic acid/chitosan polyion complex, was used, the mean score for the regeneration of newly formed bone was 2.0, and the epithelial down growth rate was 42%, thereby demonstrating both unsatisfactory regeneration of newly formed bone and unsatisfactory prevention of the epithelial down growth. Also, a considerable amount of time was required during surgery operation to completely cover the upper portion of the periodontal pocket owing to the membrane deformation.

When the medicament for treating periodontal disease of Comparative Example 4, i.e., sponge-like uncrosslinked chitin, was used, the mean score for the regeneration of newly formed bone was 1.8, and the epithelial down growth rate was 25%, thereby demonstrating both unsatisfactory regeneration of newly formed bone and unsatisfactory prevention of the epithelial down growth. Although the medicament for treating periodontal disease could easily be packed in the periodontal pocket, it was difficult to stably retain it in the pocket owing to sponge shrinkage upon blood absorption.

Those skilled in the art will recognize, or be able to ascertain using no more than routine experimentation, many equivalents to the specific embodiments of the invention described specifically herein. Such equivalents are intended to be encompassed in the scope of the following claims.

INDUSTRIAL APPLICABILITY

The medicament for treating periodontal disease of the present invention has good operability, can be easily packed to periodontal pockets of cases of high alveolar bone absorption, does not cause down growth of the epithelium owing to deficiency in mechanical strength, and thus further promotes tissue regeneration. In addition, according to this method for treating periodontal disease, the regeneration of newly formed bone becomes good, thereby suppressing the causation of down growth of the epithelium.

**Claims**

1. Use of a dry gel of a crosslinked polysaccharide prepared by crosslinking a polysaccharide with a crosslinking agent for the production of a medicament for treating periodontal disease by inserting the dry gel into a resorption portion of alveolar bone lost due to periodontal disease, whereupon the gel swells in the resorption portion.

2. Use according to claim 1, wherein the polysaccharide is an acidic polysaccharide.

3. Use according to claim 1, wherein the acidic polysaccharide is one or more compounds selected from the group consisting of alginic acid, alginic acid derivates, and salts of alginic acid or alginic acid derivates.

4. Use according to claim 1, wherein 1 to 75% of a total number of the monosaccharides constituting the polysaccharides are crosslinked.

5. Use according to claim 1, wherein the dry gel has a water concentration from 0 to 10% by weight.

6. Use according to claim 1, wherein the polysaccharide is covalently crosslinked.

7. Use according to claim 1, wherein the crosslinking agent is 2N-hydroxysuccinimide salt of diaminoethane, 2N-hydroxysuccinimide salt of N,N'-di(lysyl)-diaminoethane or 3N-hydroxysuccinimide salt of N-(lysyl)-diaminohexane.

**Patentansprüche**

1. Verwendung eines Trockengels eines vernetzten Polysaccharids, hergestellt durch Vernetzen eines Polysaccharids mit einem Vernetzungsmittel zur Herstellung eines Medikaments zur Behandlung einer parodontalen Erkrankung durch Einführen des Trockengels in einen Resorptionsbereich des Alveolarknochen, der aufgrund einer parodontalen Erkrankung abgebaut wird, woraufhin das Gel im Resorptionsbereich aufquillt.

2. Verwendung nach Anspruch 1, wobei das Polysaccharid ein saures Polysaccharid ist.

3. Verwendung nach Anspruch 1, wobei das saure Polysaccharid ein oder mehr Verbindungen sind, die aus der Gruppe bestehend aus Alginsäure, Alginsäurederivate und Salze von Alginsäure oder Alginsäurederivaten ausgewählt sind.

4. Verwendung nach Anspruch 1, wobei 1 bis 75 % einer Gesamtanzahl der Monosaccharide, die die Polysaccharide bilden, vernetzt ist.

**5.** Verwendung nach Anspruch 1, wobei das Trockengel eine Wasserkonzentration von 0 bis 10 Gew.-% hat.

**6.** Verwendung nach Anspruch 1, wobei das Polysaccharid kovalent vernetzt ist.

**7.** Verwendung nach Anspruch 1, wobei das Vernetzungsmittel 2N-Hydroxysuccinimidsalz von Diaminoethan, 2N-Hydroxysuccinimidsalz von N,N'-Di(lysyl)-diaminoethan oder 3N-Hydroxysuccinimidsalz von N-(Lysyl)-diaminohexan ist.

**Revendications**

**1.** Utilisation d'un gel sec d'un polysaccharide réticulé obtenu par réticulation d'un polysaccharide avec un agent de réticulation pour la production d'un médicament pour le traitement des parodontopathies en insérant le gel sec dans une partie de résorption de la perte d'os alvéolaire due à la parodontopathie où le gel gonfle dans la partie de résorption.

**2.** Utilisation selon la revendication 1, **caractérisée en ce que** le polysaccharide est un polysaccharide acide.

**3.** Utilisation selon la revendication 1, **caractérisé en ce que** le polysaccharide acide est un ou plusieurs composés choisis dans le groupe constitué par l'acide alginique, les dérivés acide alginique, et les sels de l'acide alginique ou les dérivés de l'acide alginique.

**4.** Utilisation selon la revendication 1, **caractérisée en ce que** 1 à 75% du nombre total de monosaccharides constituant les polysaccharides sont réticulés.

**5.** Utilisation selon la revendication 1, **caractérisée en ce que** le gel sec présente une concentration d'eau de 0 à 10% en masse.

**6.** Utilisation selon la revendication 1, **caractérisée en ce que** le polysaccharide est réticulé de manière covalente.

**7.** Utilisation selon la revendication 1, **caractérisée en ce que** l'agent de réticulation est le sel 2N-hydroxysuccinimide de diaminoéthane, le sel 2N-hydroxysuccinimide de N,N'-di(lysyl)-diaminoéthane ou le sel 3N-hydroxysuccinimide de N-(lysyl)-diaminohexane.